(19) Europäisches Patentamt
European Patent Office
Office européen des brevets

(11) **EP 3 253 378 B1**

(12) **FASCICULE DE BREVET EUROPEEN**

(45) Date de publication et mention
de la délivrance du brevet:
**08.05.2019 Bulletin 2019/19**

(21) Numéro de dépôt: **16703289.5**

(22) Date de dépôt: **04.02.2016**

(51) Int Cl.:
***A61K 9/70*** *(2006.01)* ***A61K 31/4045*** *(2006.01)*

(86) Numéro de dépôt international:
**PCT/EP2016/052376**

(87) Numéro de publication internationale:
**WO 2016/124688 (11.08.2016 Gazette 2016/32)**

(54) **SYSTÈME THÉRAPEUTIQUE TRANSDERMIQUE CONTENANT DE LA VALENTONINE ET SON UTILISATION COMME MÉDICAMENT**

TRANSDERMALES THERAPEUTISCHES SYSTEM MIT VALENTONIN UND VERWENDUNG DAVON ALS ARZNEIMITTEL

TRANSDERMAL THERAPEUTIC SYSTEM CONTAINING VALENTONIN AND USE THEREOF AS A MEDICAMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.02.2015 EP 15305161**

(43) Date de publication de la demande:
**13.12.2017 Bulletin 2017/50**

(73) Titulaire: **SOEUR JOSEFA MENENDEZ
86000 Poitiers (FR)**

(72) Inventeurs:
• **FOURTILLAN, Jean-Bernard
86000 Poitiers (FR)**
• **FOURTILLAN, Marianne
86000 Poitiers (FR)**

(74) Mandataire: **Cabinet Plasseraud
66, rue de la Chaussée d'Antin
75440 Paris Cedex 09 (FR)**

(56) Documents cités:
**WO-A1-2007/101863 US-A- 6 048 868**

**Description**

La présente invention concerne un système thérapeutique transdermique adhésif contenant à titre de principe actif une association de valentonine (VLT) et de 6-méthoxy-harmalan (6-MH) .

La présente invention concerne plus particulièrement le système thérapeutique transdermique adhésif revendiqué pour son utilisation destinée à la prévention et/ou au traitement des troubles du sommeil et des insomnies primaires, des dépressions nerveuses, des psychoses, ainsi que des maladies de Parkinson et d'Alzheimer.

On rappellera qu'il existe deux types de sommeil : le sommeil physiologique et le sommeil anesthésique. Seul, le sommeil physiologique, reconnaissable à son tracé EEG, est réparateur pour l'organisme.

Le sommeil physiologique est reconnaissable par des tracés EEG caractéristiques. Il comporte du sommeil profond à ondes lentes, réparateur, et du sommeil paradoxal qui semble important pour la mémoire.

Le sommeil physiologique survient lorsque le niveau de vigilance tombe en dessous d'un certain seuil, avec perte de conscience. Pour l'homme et les animaux à sommeil nocturne, et dans les conditions normales, par exemple en l'absence d'une dépression, durant le nycthémère, ce phénomène se produit lorsque les concentrations de la valentonine (VLT), l'hormone du sommeil, sont supérieures à celles du 6-méthoxy-harmalan (6-MH), l'hormone de la veille. Cette prévalence de la VLT sur le 6-MH existe pendant la phase d'obscurité nocturne, en France globalement entre 22h et 6h, ceci quelle que soit la saison (Fourtillan JB, Brisson A.M., Gobin P., Fourtillan M., Ingrand I., Decourt J.Ph. & Girault J., Melatonin secretion occurs at a constant rate in both young and older men and women. Am, J. Physiol. Endocrinol. Metab., 280, E11-E22 (2001)). Au cours de cette période de repos, cette prévalence de la VLT, par rapport au 6-MH, résulte des propriétés pharmacocinétiques de ces deux hormones. Chez le chien, il a été possible de déterminer les paramètres pharmacocinétiques de la VLT et du 6-MH.

Les valeurs moyennes (+ écarts types) des principaux paramètres caractéristiques des cinétiques de distribution et d'élimination des deux hormones sont présentées ci-après :

Valentonine :

$$T_{1/2}z = 0,70 (\pm 0,17) h$$

$$TMR = 0,49 (\pm 0,16) h$$

$$V_d ss = 40 (\pm 21) litres$$

$$Cl = 79,0 (\pm 19,9) litres / heures.$$

6-méthoxy-harmalan :

$$T_{1/2}z = 2,27 (\pm 0,91) h$$

$$TMR = 1,31 (\pm 0,45) h$$

$$V_d ss = 120 (\pm 51) litres$$

$$Cl = 89,19 (\pm 22,9) litres / heures.$$

Il est hautement probable que les valeurs des paramètres pharmacocinétiques, aussi bien pour les cinétiques d'élimination ($T_{1/2}z$ et Cl) de la VLT et du 6-MH, que pour leurs cinétiques de distribution (TMR et $V_d ss$), soient identiques pour l'homme et tous les animaux à sommeil nocturne, tels que le chien.

Au cours de la période de prévalence de la VLT, sur le 6-MH, le sommeil résulte de l'activation allostérique des récepteurs sérotoninergiques 5-HT$_{2C}$ par la VLT. De plus, durant le sommeil nocturne, la VLT active, par modulation allostérique, les récepteurs $\alpha_2$ adrénergiques centraux, ce qui réduit la PA et la FC pendant la période de repos, ainsi que les récepteurs D$_1$ et/ou D$_2$ dopaminergiques, avec relaxation musculaire. D'autres activations allostériques sélectives ont probablement lieu pendant la nuit.

A la fin des sécrétions, vers 6h, les concentrations de 6-méthoxy-harmalan deviennent rapidement supérieures à celles de la valentonine, notamment parce que l'élimination du 6-MH est beaucoup plus lente (T$_{1/2}$z = 2,27 h) que celle de la VLT (T$_{1/2}$z = 0,70 h). Dès lors, la prévalence du 6-MH sur la VLT a pour conséquence le blocage des récepteurs sérotoninergiques 5-HT$_{2C}$, entre 6h et 22h, l'éveil se produisant lorsque la vigilance passe au-dessus d'un certain seuil propre à chaque individu. Le blocage sélectif des récepteurs sérotoninergiques 5-HT$_{2C}$, $\alpha_2$ adrénergiques, ainsi que D$_1$ et/ou D$_2$ dopaminergiques permet ainsi, d'augmenter le niveau de vigilance, donc d'assurer l'éveil, d'augmenter la PA et la FC, et d'accroître le tonus musculaire.

Le sommeil artificiel ou anesthésique est totalement différent du sommeil naturel ou physiologique. Son tracé EEG comporte majoritairement du sommeil léger, à ondes rapides, non réparateur pour l'organisme, peu de sommeil profond. On observe un effondrement des proportions de sommeil paradoxal, en corrélation avec les effets secondaires d'amnésie antérograde, observés chez les patients.

Le sommeil artificiel ou anesthésique est par exemple induit par les benzodiazépines (Diazépam, etc.) et apparentés, (Zolpidem, Zopiclone). Tous ces composés ont en commun les propriétés pharmacologiques suivantes :

- ce sont des activateurs par modulation allostérique des récepteurs GABA$_A$-ergiques dont l'activation produit des effets neuro-inhibiteurs. Il en résulte une baisse de la vigilance à la manière d'une anesthésie, et

- en même temps, ce sont des antagonistes de la sérotonine sur les récepteurs sérotoninergiques 5-HT$_{2C}$. Ils bloquent ces récepteurs, comme le font le 6-méthoxy-harmalan, l'hormone de la veille, et le LSD. La raison de cette action est simple : ils possèdent dans leurs structures chimiques les caractéristiques pharmacophores du 6-MH.

En conséquence, malheureusement, ces composés ont deux actions pharmacologiques contradictoires :

- par leur stimulation GABA$_A$-ergique, ils produisent un mauvais sommeil, i.e ;, peu de sommeil profond, et effondrement du sommeil paradoxal, et ;
- par le blocage des récepteurs sérotoninergiques 5-HT$_{2C}$, ils s'opposent au sommeil physiologique, en agissant comme l'hormone de la veille, le 6-MH. Ils ont donc des effets dépressiogènes et anxiogènes ; ils s'opposent ainsi aux effets anxiolytiques qui résultent de leurs actions GABA$_A$-ergiques, ainsi qu'aux effets des médicaments antidépresseurs.

Il a été précédemment découvert (FR 2 898 358) que pendant le temps de sommeil nocturne (entre 22h, début de la sécrétion, et 6h fin de la sécrétion), et quelle que soit la saison, la mélatonine produite par la glande pinéale, suite à une première acétylation de la sérotonine, facilitée par des N-acétyltransférases, subit, dès sa production, une seconde étape d'acétylation enzymatique par des N-acétyltransférases, donnant successivement deux dérivés de bêta-carboline, à savoir le 6-méthoxy-1-méthyl-3,4-dihydro-bêta-carboline, appelée 6-méthoxy-harmalan (6-MH), et la 2-acétyl-6-méthoxy-1-méthylène-3,4-dihydro-bêta-carboline, appelée valentonine (VLT).

Ainsi le système veille-sommeil fonctionne avec ces trois hormones : la mélatonine (MLT), la valentonine (VLT) et le 6-méthoxy-harmalan(6-MH). La mélatonine étant le précurseur de la VLT et du 6-MH, elle en est aussi le marqueur. C'est ainsi qu'en mesurant les concentrations plasmatiques de MLT, on peut connaître l'état du système chez des sujets. Des études pharmacocinétiques de la sécrétion pinéale (Am.J.Physiol.Endocrinol.metab., 280, E11-E22) chez 12 sujets volontaires jeunes et 12 sujets volontaires âgés, ont montré une grande variabilité dans les vitesses de sécrétion, donc dans les quantités sécrétées, de MLT dans des proportions de 1 à 10, selon les sujets. C'est sans doute, cette grande variabilité qui est à l'origine des nombreuses affections d'origine neurologique observées chez l'homme, telles que l'insomnie primaire, les troubles du sommeil, les dépressions réactionnelles et endogènes, les affections neurodégénératives (Parkinson, Alzheimer), et les troubles psychotiques.

Le traitement des dépressions, des états psychotiques, de la maladie de parkinson, et de la maladie d'Alzheimer doivent impliquer la compréhension de ce même système veille-sommeil. Les explications des mécanismes de ces troubles, aussi bien que les modes d'action de leurs nouveaux traitements ne connaissaient pas le mécanisme du sommeil physiologique. On pensait en effet, qu'un excès de dopamine était à l'origine des psychoses, alors que l'on vient de découvrir que c'est un excès du 6-méthoxy-harmalan, l'hormone de la veille, qui est à l'origine des états psychotiques. La dépression, tout au moins réactionnelle, n'est pas une maladie incurable, car on en guérit toujours, en renforçant le système veille-sommeil. Les causes des maladies neurodégénératives, telles que les maladies de Parkinson et d'Alzheimer, proviennent en réalité d'un déficit quantitatif du système veille-sommeil.

Ainsi, ces maladies pourront sans doute être prévenues et stoppées dans leur progression. Dans la maladie d'Alzheimer, le sommeil et la cognition pourront être restaurés par administration d'un valentonergique et de 6-méthoxy-harmalan.

Le système [(VLT) - (6-MH)] joue donc un rôle essentiel en assurant la régulation des vies psychique et végétative de l'organisme pendant les 24 heures du nycthémère.

Le fonctionnement harmonieux de ce système est directement relié :

- aux structures chimiques des hormones du sommeil (VLT) et de la veille (6-MH), qui leur permettent de stimuler (VLT), pendant la période de repos nocturne, ou de bloquer de façon sélective (6-MH), les récepteurs mis en jeu dans la régulation des organes et processus physiologiques qui fonctionnent différemment pendant les périodes de repos et d'activité ;

- aux propriétés pharmacocinétiques de la valentonine et du 6-méthoxy-harmalan, qui permettent d'assurer la prévalence de la VLT, pendant les 8 heures de la période de sommeil, entre 22h et 6h, et la prévalence du 6-MH, pendant la période de veille ou d'activité, entre 6h et 22h ;

- à l'évolution des concentrations de la VLT et du 6-MH, correspondant à une infusion à vitesse constante dans la circulation sanguine, pendant les 8 heures de la sécrétion des deux hormones par la glande pinéale.

La demanderesse a donc cherché à mettre en oeuvre, avec le système thérapeutique transdermique revendiqué, pour son utilisation destinée à une thérapeutique hormonale substitutive à base de VLT, en combinaison avec le 6-MH, dans le but de réguler le fonctionnement de l'organisme suivant le profil de chaque sujet qui peut être déterminé par une méthode de dosage des concentrations plasmatiques de mélatonine dans un échantillon sanguin prélevé entre 22h et 6h du matin, de préférence à partir de 1h du matin.

La valentonine ne pouvant, comme la plupart des composés endogènes, être administrée par voie orale, la présente invention concerne un système thérapeutique transdermique d'une ou des deux hormones précitées, avantageusement sous forme de patchs comportant un ou deux réservoirs pour traiter les affections d'origine neurologique, qui résultent d'un déficit ou d'un excès quantitatif du système [(VLT)-(6-MH)].

Ainsi l'invention se rapporte à un système thérapeutique transdermique qui contient à titre de principe actif une association de valentonine (VLT) et de 6-méthoxy-harmalan (6-MH), qui peut se présenter de manière connue en soi, sous la forme d'un patch de type réservoir à un ou deux compartiments ou bien de type matriciel.

De préférence, l'invention concerne un système thérapeutique transdermique adhésif contenant une association d'une charge de valentonine (VLT) et de 6-méthoxy-harmalan (6-MH) apte à délivrer dans la circulation sanguine la valentonine et de 6-méthoxy-harmalan dans une proportion massique [VLT] / [6-MH] égale à 4 pendant la durée d'application du patch, de préférence pendant environ 8h à 10h.

La réalisation pratique des patchs sera déterminée par l'homme du métier en application de ses connaissances générales en la matière pour obtenir une administration systémique contrôlée et prolongée de la valentonine et du 6-méthoxy-harmalan, sur toute la période d'application du patch, c'est-à-dire pendant une durée d'environ 8h à 10h.

De manière générale, en faisant usage de ses connaissances générales en la matière, l'homme du métier pourra intégrer les deux principes actifs dans deux patchs indépendants, mélanger les deux principes aussi bien dans une même matrice que dans des matrices superposées, ou encore dans des matrices montées de façon adjacente sur un même film de protection. Bien entendu, la valentonine et le 6-méthoxy-harmalan peuvent également être présents dans un autre type de patchs à deux réservoirs indépendants.

On rappellera de manière générale qu'il existe deux grandes classes de système thérapeutique transdermique ou patch, qui sont toutes deux constituées :

- d'une couche externe imperméable,
- d'un compartiment renfermant le principe actif,
- d'un organe de contrôle de la libération du ou des principe(s) actif(s),
- d'une pièce adhésive permettant le maintien du patch sur la zone d'application de la peau, ainsi que
- d'un support protecteur destiné à être retiré juste avant l'application du patch.

Le patch selon la présente invention pourra donc être réalisé avec une conformation, soit de type réservoir, soit de type matriciel.

Le type de patch réservoir comportera un ou deux réservoir(s) séparé(s) contenant le ou les principe(s) actif(s) en solution ou en suspension dans la matrice polymère venant au contact de la peau par l'intermédiaire d'une membrane polymérique semi-perméable permettant d'ajuster la vitesse de libération du ou des principe(s) actif(s).

Le patch de type matriciel comportera une masse polymérique à l'intérieur de laquelle le ou les principe(s) actif(s) se trouveront dissous ou dispersés dans les proportions adéquates. La libération de ces principes actifs se fait par diffusion au travers des chaînes polymères de ladite matrice.

Selon un mode de réalisation particulier de ce type de patch, l'adhésif recouvre la totalité de la surface de libération de la matrice et fait partie intégrante de cette dernière. On se trouve ainsi en présence d'un patch de type adhésif actif bien connu de l'homme du métier qui relève d'une fabrication simplifiée et qui permet des réalisations de patchs de faible épaisseur et de souplesse appropriée permettant une application confortable sur la peau du patient.

Dans un mode de réalisation avantageux de la présente invention, la valentonine et le 6-méthoxy-harmalan sont disposés dans des réservoirs distincts l'un de l'autre, ce qui permet d'écarter tout problème de stabilité ou tout autre risque d'interactions susceptibles d'altérer les flux de diffusion des principes actifs l'un par rapport à l'autre lorsqu'ils sont conditionnés en mélange dans une même matrice.

Dans un mode de réalisation particulier de l'invention d'un patch bi-compartimenté, chaque compartiment du patch peut contenir une matrice polymère renfermant isolément chacun des deux principes actifs. Pour assurer une bonne infusion de ces principes actifs dans la circulation sanguine à la dose appropriée, l'homme du métier pourra aisément fixer les paramètres suivants :

- le rapport des surfaces et des volumes de chacun des compartiments du patch ;
- l'ajout éventuel d'un additif hydrophile ;
- l'ajout éventuel d'un promoteur d'absorption, et ;
- de façon plus générale, tout type d'additifs bien connus de l'homme du métier permettant de bien contrôler les flux de la valentonine et du 6-méthoxy-harmalan.

De manière en soi connue, un tel patch comprend un film protecteur pelable qui est destiné à préserver la face adhésive à appliquer sur la peau après la fabrication du patch et durant toute sa durée de stockage. De manière en soi connue, l'homme du métier aura par exemple recours à des films polyesters dont l'une des faces peut être traitée par des silicones anti-adhérentes.

La matrice polymère adhésive renfermant la valentonine et le 6-méthoxy-harmalan, peut être de type polymère ou copolymère, par exemple de type polyisobutylène ou polyacrylique, copolymère d'éthylène et d'acétate de vinyle ou encore de type polymère de silicone.

On utilisera de façon habituelle des résines acryliques autoréticulables par exemple de type Duro-Tak® commercialisé par la société Henkel.

Comme indiqué précédemment, des promoteurs d'absorption peuvent être utilisés de manière en soi connue. A titre d'exemple de tels additifs, on mentionnera les éthers monoalkyliques de diéthylèneglycol, les glycérides polyglycolisés saturés, le propane-diol 1,2 ou encore l'éthanol.

La valentonine et le 6-méthoxy-harmalan ayant un caractère lipophile, il peut également s'avérer nécessaire d'introduire dans la matrice polymère des additifs hydrophiles tels que des polymères naturels ou synthétiques tels que les gommes guar, la gomme de xanthane ou encore la polyvinylpyrrolidone.

Selon un mode de réalisation particulier de la présente invention, la couche adhésive contenant les principes actifs, objet de la présente invention contient un ou plusieurs copolymères à base d'alkyl(méth)acrylate et en particulier l'éthyl méthacrylate, le n-octyl méthacrylate et le dodécyl méthacrylate. D'autres monomères peuvent être ajoutés à ce copolymère alkyl(méth)acrylate et en particulier des monomères acrylonitriles, vinylacétate, vinylproprionate ou encore 1-vinyl-2-pyrrolidone.

Pour assurer la stabilité des principes actifs au sein des matrices du patch et en particulier éviter tout risque de dégradation par oxydation durant le stockage, on ajoutera également de façon avantageuse de faibles proportions d'un agent antioxydant tel que l'a-tocophérol et le palmitate d'ascorbyle.

Dans le cadre de la présente invention, la Demanderesse a pu déterminer que les indications principales de l'administration de valentonine seule ou en association avec le 6-MH peuvent être classées en deux grandes catégories :

A) Affections provenant d'un déficit quantitatif du système [(VLT)-(6-MH)] :

- les insomnies primaires ;
- les troubles du sommeil ;
- les dépressions endogènes et réactionnelles, et ;
- les affections neurodégénératives (Parkinson, Alzheimer, etc) .

Il apparaît clairement que les affections dues à un déficit du système [(VLT)-(6-MH)] peuvent être traitées par une association de valentonine biodisponible pour la voie d'administration choisie, et du 6-méthoxy-harmalan.

Les propriétés pharmacocinétiques de la valentonine et du 6-MH, et, en particulier, leurs biodisponibilités, pour la voie d'administration choisie, doivent permettre, étant donné qu'il s'agit d'un traitement hormonal substitutif, comportant deux hormones, de reproduire, le mieux possible, les courbes d'évolutions des concentrations plasmatiques des deux hormones du système [(VLT)-(6-MH)], pendant 24 h.

Pour un traitement hormonal substitutif, comportant deux hormones (VLT et 6-MH), dont l'une, la valentonine est instable puisque totalement hydrolysée en milieu gastrique acide, la voie d'administration retenue dans le cadre de la présente invention est la voie transdermique pour les raisons suivantes :

1) De même que la voie intraveineuse (beaucoup trop contraignante, car, pour reproduire les courbes physiologiques d'évolutions des taux plasmatiques des deux hormones, elle nécessiterait une perfusion IV, du mélange des deux hormones, pendant les 8 heures du repos nocturne, entre 22 h et 6 h), la voie transdermique permet d'administrer simultanément les deux hormones naturelles, la valentonine et le 6-méthoxy-harmalan, sous forme d'un seul et même patch transdermique. Pour le traitement des affections consécutives à un déficit quantitatif du système [(VLT)-(6-MH)], il peut, ainsi, être envisagé d'appliquer un patch pendant les 8 heures de la période de sécrétion pinéale des deux hormones, entre 22 h et 6 h, dans les proportions appropriées.

2) De plus l'utilisation de patchs ayant deux réservoirs distincts (un réservoir pour chaque hormone) devrait permettre de contrôler séparément les libérations des deux hormones ; sans qu'elles n'interfèrent, l'une sur l'autre, sur leurs biodisponibilités et sur la labilité de la combinaison des deux produits mis en contact.

Dans le cas de l'application aux insomnies primaires et affections analogues, on observe une insuffisance de la sécrétion de valentonine (VLT), et, parallèlement, du 6-méthoxy-harmalan (6-MH), qui sont consécutives à une sécrétion pinéale de la mélatonine trop faible. En pareil cas, un dosage de la mélatonine, en tant que marqueur des deux hormones du système [(VLT)-(6-MH)] dans le plasma, au cours de la nuit, permettra de déterminer quantitativement le déficit, et d'adapter en conséquence les dosages de la valentonine et du 6-MH dans l'association.

Pour les autres indications, telles que les troubles du sommeil, les dépressions, les maladies neurodégénératives (Parkinson, Alzheimer), il apparaît également nécessaire d'administrer des associations de valentonine et du 6-méthoxy-harmalan. Ces pathologies correspondent à un déficit quantitatif du système VLT/6-MH. L'importance de ce déficit varie avec l'affection. Il peut être évalué par dosage de la mélatonine à effectuer au plateau avant 6h du matin, en tant que marqueur du système [(VLT)-(6-MH)].

Il convient de rappeler que dans les conditions physiologiques, les deux hormones du système [(VLT)-(6-MH)] et la mélatonine, sont délivrées par la glande pinéale, à la circulation sanguine, pendant 8 heures, entre 22 h et 6 h, selon une perfusion intra veineuse à vitesse constante, i.e. avec une cinétique d'ordre 0. L'évolution des concentrations des deux hormones dans le plasma sanguin, dépend de leurs propriétés pharmacocinétiques, et en particulier de leurs cinétiques d'élimination caractérisées par leurs temps de demi-vie d'élimination terminale $T_{1/2}z$. Ainsi, pour chaque hormone (VLT et 6-MH) sécrétée par la glande pinéale de façon variable pour chaque sujet, l'évolution des concentrations plasmatiques dépend :

- de la vitesse de sécrétion, qui reflète, pour chaque sujet, et d'un point de vue quantitatif, le niveau de son système [(VLT)-(6-MH)]. Cette donnée est propre à chaque sujet. La valeur de cette donnée individuelle varie avec l'affection d'origine neurologique dont est victime chaque patient, et ;
- des caractéristiques pharmacocinétiques des deux hormones. Il est en effet hautement probable que les cinétiques d'éliminations et de distributions sont identiques chez l'homme et tous les animaux à sommeil nocturne, tels que le chien pour lequel les demi vies $T_{1/2}z$ sont respectivement égales à 0,70 h et 2,27 h, pour la valentonine et le 6-méthoxy-harmalan, ainsi que les volumes de distribution sont respectivement égaux à 5 litres/kg et 15 litres/kg pour la VLT et le 6-MH.

On peut donc prévoir, pour le traitement des affections provenant d'un déficit du système [(VLT)-(6-MH)], des dosages de valentonine et de 6-méthoxy-harmalan différents, dans les deux réservoirs des patchs, selon le type d'affection et les taux de mélatonine observés chez les patients.

B) L'invention s'étend aussi au traitement des affections provenant d'un excès quantitatif du système [(VLT)-(6-MH)] ; il s'agit en particulier des états psychotiques.

Le traitement des troubles psychotiques, dus à un excès de 6-méthoxy-harmalan, entre 6 h et 22 h, au cours de la journée, peut être envisagé par déplacement de l'excès de 6-MH, par la valentonine, durant cette période.

Dans le cadre de la présente invention, ont été déterminées les doses de valentonine (VLT) et de 6-méthoxy-harmalan (6-MH) dans les associations des deux hormones, administrées par voie transdermique.

Les affections d'origine neurologique les plus courantes (insomnies primaires, troubles du sommeil, dépressions nerveuses réactionnelles et endogènes, et affections neurodégénératives du type Parkinson et Alzheimer) sont dues à des déficits quantitatifs du système [(VLT)-(6-MH)] qu'il faudra restaurer.

La présente invention vise donc un système thérapeutique transdermique adhésif, selon les revendications, pour son utilisation dans le traitement des affections dues à des déficits quantitatifs des hormones du système [(VLT)-(6-MH)]

Pour assurer et maintenir, de façon harmonieuse, et à l'identique de la physiologie, une bonne régulation des vies psychique et végétative de l'organisme, pendant les 24h du cycle nycthéméral, il est impératif d'administrer l'association (VLT+6-MH) sous une forme susceptible de reproduire la sécrétion pinéale des deux hormones entre 22h (ou au coucher du patient) et 6h (ou au lever du patient) . Ce qui impose de respecter les impératifs suivants :

1. Administration des hormones physiologiques, la valentonine et le 6-méthoxy-harmalan, et non pas de leurs succédanés de synthèse. En effet, chimiquement différents, les succédanés de synthèse ont des propriétés pharmacocinétiques différentes qui rendraient impossible l'alternance des périodes de prévalence de chaque hormone, aux moments précis des fins des périodes de repos (6h, ou heure du lever) et d'activité (22h, ou heure du coucher). Cet impératif nécessite des profils pharmacocinétiques très précis et concordants pour les deux hormones naturelles (VLT et 6-MH). Ceci d'autant plus que ces prévalences de concentrations doivent exister pendant les deux périodes, non seulement au niveau plasmatique, mais aussi aux niveaux des sites d'action des deux hormones (récepteurs $5\text{-HT}_{2C}$, $\alpha_2$, $D_1$ et $D_2$, etc) .

2. Le mode d'administration doit reproduire une infusion des deux hormones à vitesse constante et pendant 8 heures (entre 22h, au coucher, et 6h au lever). Une perfusion endoveineuse, pendant 8 heures étant trop contraignante, et la VLT étant totalement dégradée en milieu gastrique acide, la voie extravasculaire susceptible de réaliser une telle infusion dans le cadre de la présente invention est la voie transdermique, sous forme de patchs à deux réservoirs, appliqués pendant 8 heures, au coucher et enlevés au lever.

3. Les calculs des doses de VLT et 6-MH, biodisponibles qui doivent être libérées dans l'organisme à partir du patch, peuvent être effectués pour chaque affection traitée, à partir des valeurs des taux plasmatiques de mélatonine mesurés entre 1h et 6h du matin chez les patients à traiter.

Au cours d'une étude de la cinétique de la sécrétion de mélatonine par la glande pinéale réalisée chez l'homme, dans deux groupes de 12 sujets volontaires sains des deux sexes, jeunes et âgés (Fourtillan JB, Brisson A.M., Gobin P., Fourtillan M., Ingrand I., Decourt J.Ph. & Girault J., Melatonin secretion occurs at a constant rate in both young and older men and women. Am, J. Physiol. Endocrinol. Metab., 280, E11-E22 (2001)), on a pu mesurer les quantités totales de mélatonine (MLT) sécrétées pendant la nuit (entre 22h et 6h). On a observé une variabilité interindividuelle importante (de 1 à 10) chez les 24 sujets. Cette étude a montré que la quantité totale de MLT sécrétée, pendant les 8h de la nuit, s'élevait en moyenne à 28,7 $\mu$g (35,7 $\mu$g chez les hommes jeunes adultes sains de poids moyen égal à 74 kg, soit 0,48 $\mu$g par kg ; et 21,6 $\mu$g chez les femmes jeunes adultes saines de poids moyen égal à 54 kg, soit 0,40 $\mu$g par kg) pour un poids moyen égal à 64 kg, soit une sécrétion totale de MLT égale à 0,44 $\mu$g par kg. Par ailleurs, chez ces 12 sujets jeunes, les quantités totales de MLT sécrétées pendant la nuit varient de 10 à 60 $\mu$g, correspondant à des concentrations plasmatiques maximales, observées à partir de 1h du matin, comprises entre 19 et 93,7 picogrammes par ml, avec une valeur moyenne égale à 54,5 picogrammes par ml. Chez les 12 sujets âgés, les concentrations plasmatiques maximales observées à partir de 1h du matin, étaient comprises entre 9,6 et 124,7 picogrammes par ml, avec une valeur moyenne égale à 45,6 picogrammes par ml. En moyenne, les concentrations plasmatiques maximales de MLT sont plus faibles chez les personnes âgées, bien que la valeur limite maximale (124,7 pg/ml) soit observée chez une personne âgée.

Cette étude permet de situer le niveau moyen de la quantité totale de mélatonine, sécrétée par la glande pinéale pendant la nuit (durée moyenne égale à 8h, quelle que soit la saison), à environ 30 $\mu$g, soit 0,5 $\mu$g par kg, chez les sujets jeunes adultes sains des deux sexes. Il ne faut pas perdre de vue qu'il existe une variabilité interindividuelle très importante pour ce paramètre biologique. En effet, cette sécrétion varie de 10 à 60 $\mu$g dans l'échantillon des 12 sujets volontaires jeunes adultes sains (6 hommes, 6 femmes) correspondant à des concentrations plasmatiques maximales observées à partir de 1h du matin comprises entre 19 et 93,7 picogrammes par ml.

Il n'est pas possible de mesurer les sécrétions pinéales nocturnes de MLT chez les patients, mais cette étude, réalisée chez des personnes jeunes et âgées, montre que chez les 24 sujets étudiés, les concentrations plasmatiques maximales de mélatonine observées à partir de 1h du matin, varient de 9,6 à 124,7 picogrammes par ml. Ces taux sont le reflet des sécrétions pinéales totales de MLT.

Il faut retenir de cette étude que la sécrétion pinéale totale de mélatonine s'élève en moyenne à 30 $\mu$g avec des valeurs comprises entre 10 et 60 $\mu$g chez les sujets jeunes adultes sains. Ces sécrétions correspondent à des concentrations plasmatiques maximales, observées à partir de 1h du matin, comprises entre 19 et 94 picogrammes par ml. En extrapolant cette corrélation entre les sécrétions pinéales totales et les concentrations plasmatiques maximales de mélatonine, aux sujets âgés de l'étude, on peut énoncer les conclusions suivantes :

- Les sécrétions totales de mélatonine par la glande pinéale, pendant la nuit (durée 8h, entre 22h et 6h) varient de 5 $\mu$g à 100 $\mu$g ;
- Les concentrations plasmatiques maximales de mélatonine observées à partir du 1h du matin, sont le reflet de cette sécrétion ; elles varient entre 10 et 125 picogrammes de mélatonine, autour d'une valeur moyenne voisine de 50 picogrammes par ml ;

- La mesure des concentrations plasmatiques de mélatonine, à partir de 1h du matin chez les patients, permettra de connaître l'importance de leur sécrétion pinéale de MLT, et par voie de conséquence, l'état du système [(VLT)-(6-MH)], chez ces patients puisque la MLT est le bioprécurseur des deux hormones, la valentonine et le 6-méthoxy-harmalan.

3.1 Corrélations entre les sécrétions de mélatonine et les affections d'origine neurologique dues à un déficit du système [(VLT)-(6-MH)].

Malgré l'absence dans la littérature scientifique et pour des raisons techniques, de données concernant l'importance de la sécrétion pinéale de mélatonine au cours des différentes affections d'origine neurologique, qui semblent lui être reliées, il est possible d'établir une corrélation entre les sécrétions pinéales de MLT et ces affections. L'évaluation de la sécrétion pinéale de MLT peut être faite, comme nous venons de le montrer, par un dosage de la concentration plasmatique maximale de mélatonine à partir de 1h du matin, chez les patients. On peut édicter les correspondances suivantes :

- des concentrations plasmatiques maximales de MLT inférieures à 10 pg/ml, correspondant à des sécrétions pinéales de MLT inférieures à 5 $\mu$g, sont observées dans les insomnies primaires, et les affections neurodégénératives telles que la maladie de Parkinson. Dans le cas particulier de la maladie d'Alzheimer, les rares études qui ont été réalisées montrent un effondrement de la sécrétion pinéale de MLT, avec des concentrations de MLT dans les milieux biologiques inférieures aux limites de détection des techniques d'analyse de la MLT ;
- des concentrations plasmatiques maximales de MLT comprises entre 10 pg/ml et 50 pg/ml, correspondant à des sécrétions pinéales de MLT comprises entre 5 $\mu$g et 25 $\mu$g, sont observées dans les troubles du sommeil, ainsi que dans les dépressions nerveuses endogènes et réactionnelles.

Ainsi, la valeur de la concentration plasmatique maximale de MLT, reflet de l'importance de la sécrétion pinéale de MLT, et par voie de conséquence des sécrétions pinéales des deux hormones du système [(VLT)-(6-MH)], peut venir compléter le diagnostic clinique de l'affection neurologique, et sert de base à l'établissement des dosages des hormones dans l'association (VLT + 6-MH) qu'il faut administrer par voie transdermique.

Produits chimiques

La mélatonine a été synthétisée par Cerillant et achetée auprès de Promochem (Molsheim, France) et l'étalon interne, la D4-mélatonine, a été synthétisé par CDN Isotopes et acheté auprès de C.I.L (Sainte-Foix-La-Grande, France). Les cartouches pour SPE Oasis® HLB, 1 m1, 30 mg ont été achetées auprès de Waters (Milford, MA, États-Unis). Le méthanol HPLC Analyzed, l'eau HPLC Analyzed, l'acide formique à 98-100 % pour Analyzed, l'acétonitrile Ultra Gradient Grade HPLC Analyzed, le chlorure de méthylène HPLC Analyzed, la solution 2N d'hydroxyde de sodium et l'acétate de sodium ont été obtenus auprès de Merck AG (Darmstadt, Allemagne). Tous les solvants étaient de haute pureté et ils ont été utilisés sans autre purification. Afin d'éviter toute contamination ultérieure des échantillons : des tubes en verre jetables de 3,5 ml, 10 ml et des tubes en verre jetables de 10 ml avec des bouchons en Téflon ont été utilisés pour cette analyse.

Courbes d'étalonnage

Des solutions mères de mélatonine et d'étalon interne ont été préparées par dissolution de chacun des composés de référence purs dans du méthanol afin d'obtenir une concentration primaire de 1000 ng.$\mu$l$^{-1}$. Ces solutions ont été stockées à -20°C jusqu'à utilisation. Les solutions de travail des étalons, préparées pour l'analyse, ont été obtenues par des dilutions appropriées des solutions mères dans du méthanol. Elles ont été stockées à moins de 20°C jusqu'à utilisation.

Différents jeux de plasma témoin ont été testés pour déterminer les concentrations de mélatonine endogène.

Une courbe d'étalonnage à 9 points a été préparée tous les jours en chargeant des aliquotes (1 ml) de plasma humain dépourvu de substance médicamenteuse avec 40 $\mu$l de la solution d'étalon interne à 5 pg.$\mu$l$^{-1}$ (200 pg de $D_4$-mélatonine) et diverses quantités de mélatonine situées dans la plage de 1 à 200 $\mu$g. Les échantillons de blanc ont été préparés de façon similaire en chargeant 1 ml de plasma témoin uniquement avec la solution d'étalon interne.

Extraction à partir des échantillons de plasma

Extraction en phase solide

Toutes les expériences de SPE ont été réalisées en utilisant une colonne Waters Oasis® HLB 1 ml, 30 mg. La colonne a été conditionnée avec 1 ml de méthanol, suivi de 2 ml d'eau. Du plasma (1 ml), fortifié comme il a été indiqué ci-dessus avec la solution de D4-mélatonine, a été déposé dans un tube en verre de 3,5 ml et dilué avec 1 ml d'eau. Après une brève agitation en utilisant un mélangeur vortex, l'échantillon dilué a été chargé sur la colonne. Après le lavage de la colonne avec 1 ml d'eau, les composants ont été élués avec 1 ml de méthanol. Les échantillons ont été évaporés jusqu'à l'état sec sous un courant doux d'azote et dissous dans 1 ml d'hydroxyde de sodium 0,002N dans de l'eau.

Extraction en phase liquide

Après une brève agitation en utilisant un vortex, 6 ml de chlorure de méthylène ont été ajoutés. La procédure d'extraction a été conduite sur une période de 10 min en utilisant un agitateur alternatif. Les tubes ont été ensuite centrifugés à 4000 tr/min pendant 10 min. La couche aqueuse supérieure a été totalement éliminée et la phase organique restante a été transférée dans un tube en verre de 10 ml et évaporée jusqu'à l'état sec à 40°C sous un courant doux d'azote. Le résidu sec a été redissous dans 200 $\mu$l d'eau et conservé à +4°C avant l'injection. Un volume fixe de 75 $\mu$l a été injecté dans le système de LC-MS/MS.

Procédure de validation

Durant cette procédure de validation, trois courbes d'étalonnage de plasma ont été préparées et exécutées le même jour par le même analyste. Les paramètres de régression ont été calculés pour évaluer la linéarité et la reproductibilité de la technique. En outre, pour estimer la précision et l'exactitude au cours de la même journée et d'une journée à l'autre du procédé, des tests de répétabilité ont été réalisés à des concentrations différentes (1, 2, 25 et 200 pg.ml$^{-1}$) sur trois jours distincts. Les échantillons de plasma chargés ont été analysés sur une période de 1 semaine par deux opérateurs différents et, pour chaque concentration, l'écart-type relatif et le pourcentage moyen d'erreur ont été calculés.

La limite de quantification (LLQ), qui doit être déterminée avec un niveau de confiance indiqué, a été définie comme la concentration détectable la plus basse produisant un signal significativement supérieur au signal moyen mesuré dans des échantillons témoins représentatifs. En premier, pour calculer la LLQ du procédé, un test de répétabilité a été réalisé avec 5 échantillons de plasma, les blancs, dépourvus de substance médicamenteuse. Le signal moyen (Ybl) observé au temps de rétention de la mélatonine et l'écart-type associé (Sbl) ont été utilisés pour calculer une valeur théorique (Yth) de la limite de quantification. Ensuite, un test de répétabilité a été réalisé avec 5 répliques des échantillons de plasma chargés avec une concentration de mélatonine proche de cette limite de quantification théorique. La valeur moyenne du signal (Yloq) a été comparée statistiquement au signal moyen (Ybl) obtenu avec les échantillons témoins. Après avoir testé l'homogénéité de la variance (p < 0,05), un test t de Student ou un test de Welch a été utilisé afin de démontrer que Yloq était significativement supérieure à Ybl au niveau de probabilité de 97,5 %.

Pour déterminer la stabilité de la mélatonine dans le plasma humain congelé, des solutions de travail ont été ajoutées à un lot de plasma humain, blanc, et ensuite stockées en dessous de la température de congélation de -20°C. Six échantillons de plasma chargés avec 2 et 200 pg.ml$^{-1}$ de mélatonine seront extraits et analysés avant et après deux et trois cycles de congélation/décongélation. Entre chaque cycle, les échantillons seront maintenus dans des conditions de température de stockage identiques (approximativement -20°C) à celles utilisées pour une analyse normale des échantillons.

Les extraits de plasma seront analysés de façon répétée durant la période de validation afin de vérifier la dégradation possible des composés lors d'un stockage dans le solvant à approximativement +4°C. L'évaluation de la stabilité est basée sur les concentrations rétrocalculées qui devront se situer à ± 15 % (sauf pour LLQ ± 20 %) et une forme de pic chromatographique acceptable.

Afin de calculer la récupération globale du procédé, une courbe d'étalonnage de plasma (de la limite inférieure de quantification jusqu'à la limite supérieure de quantification) sera préparée et analysée. Dans le même temps, six (6) échantillons chargés avec 2, 25 et 200 pg.ml$^{-1}$ de mélatonine seront extraits et analysés. Les valeurs moyennes de la réponse seront calculées et comparées à celles obtenues avec les extraits de plasma blancs (n = 6) préparés et chargés avec l'étalon pur de concentrations équivalentes à la fin du traitement des échantillons. La récupération de l'étalon interne sera également déterminée à la concentration utilisée pour charger les échantillons.

Analyse LC-MS/MS

Instrument

Le système de CLHP était constitué d'un système de la série 1100 de Agilent doté d'une pompe binaire et d'un auto-échantillonneur thermostaté.

Le système de LC-MS/MS était constitué d'un spectromètre de masse en mode tandem triple quadripolaire API 4000 (APPLIED BIOSYSTEMS) fonctionnant en mode d'ionisation positif Turbo IonSpray. Ce système était couplé à la sortie de la colonne de CLHP en utilisant une longueur de tuyau PEEK.

Conditions chromatographiques

La chromatographie a été réalisée sur une colonne C18 (3,5 $\mu$m, 50 x 2,1 mm de D.I., XBridge™ Waters, Milford, MA, États-Unis). La phase mobile était : acétonitrile-5 mM d'acétate d'ammonium aqueux-acide formique (25/75/0,01). Le débit était de 0,2 ml/min.

Conditions de spectromètre de masse

Le spectromètre de masse a fonctionné en mode d'ionisation positif Turbo IonSpray à 500°C. Les échantillons ont été analysés par le suivi de réactions sélectionnées (SRM) en employant la transition de l'ion du précurseur [M + H]$^+$ en l'ion du produit pour l'analyte et l'étalon interne. Les ions suivis dans le mode de suivi des réactions (MRM) étaient m/z 233,18 (ion du précurseur : [M+H]$^+$) à 174,10 (ion du produit) pour la mélatonine et m/z 237,22 (ion du précurseur : [M+H]$^+$) à 178,10 (ion du produit) pour la D$_4$-mélatonine (E.I.) . Les ions des produits formés sont obtenus par perte de fragment [NH-CO-CH$_3$].

> La tension de l'ionisation a été réglée à + 4,8 KV.
> Le gaz nébuliseur du TIS (gaz 1) a été réglé sur 40.
> Le gaz du dispositif de chauffage du TIS (gaz 2) a été réglé sur 50.
> Le gaz rideau a été réglé sur 12.
> Le gaz de dissociation induite par collision (CAD) a été réglé sur 10.
> Les valeurs de DP, EP, CE et CXP ont été réglées, respectivement, sur 50, 10, 17 et 10.
> La durée de temporisation a été réglée sur 500 ms pour chaque composé.

RESULTATS ET DISCUSSION

Prétraitement et analyse des échantillons

L'analyse quantitative d'un composé présent à une concentration très faible de l'ordre du femtogramme dans un liquide biologique complexe représente un défi colossal pour l'analyste en charge d'un lot important d'échantillons à analyser quotidiennement. En premier lieu, les échantillons plasmatiques doivent être dépourvus de la plupart des substances endogènes qui pourraient interférer durant la détermination de la mélatonine.

Nous avons observé qu'une procédure d'extraction solide-liquide suivie d'une procédure d'extraction liquide-liquide, en utilisant un solvant de haute pureté comme le chlorure de méthylène en phase aqueuse alcaline, a mené à un résidu propre dépourvu de la plupart des impuretés. Le procédé d'extraction a conduit à un gain de temps considérable, si bien que le débit du laboratoire a été augmenté de plus de 100 échantillons plasmatiques par jour. Le résidu dissous dans l'eau, qui est très protecteur pour la colonne, a permis de traiter quotidiennement un grand nombre d'injections grâce à un temps court d'exécution chromatographique (4,5 min). En utilisant nos conditions analytiques, le temps de rétention de la mélatonine et de la D$_4$-mélatonine a été d'environ 2,3 min. Les deux pics chromatographiques avaient une forme gaussienne et la largeur du pic à la base de chaque analyte était inférieure à 30s. La largeur du pic calculée à mi-hauteur était d'environ 5s. Une durée de temporisation de 500 ms par plage de masses a mené à un minimum de 20 points de donnée pour la mesure de chaque composé, en prenant en compte le temps d'inactivité total du système.

Récupération

L'efficacité de l'extraction a été d'environ 80 % et ces résultats ont été confirmés par la suite durant la procédure de validation de la CLHP-MS/MS.

Récupération globale calculée d'après les résultats obtenus après l'analyse des répliques d'un extrait de matrice biologique et d'un étalon non traité chargés avec 200 (pg.ml$^{-1}$) de mélatonine :

| Echantillon n° | Rapport de surface de pic de l'extrait de matrice biologique | Etalon non traité |
|---|---|---|
| 1 | 1,407 | 1,761 |
| 2 | 1,433 | 1,793 |
| 3 | 1,442 | 1,748 |
| 4 | 1,404 | 1,754 |
| 5 | 1,499 | 1,698 |
| 6 | 1,346 | 1,796 |
| Moyenne | 1,422 | 1,758 |
| ET | 0,050 | 0,036 |
| n | 6 | 6 |
| ETR (%) | 3,55 % | 2,03 % |
| Valeur minimale | 1,346 | 1,698 |
| Valeur maximale | 1,499 | 1,796 |
| Récupération globale = 80,85 % | | |

Récupération globale calculée d'après les résultats obtenus après l'analyse des répliques d'un extrait de matrice biologique et d'un étalon non traité chargés avec 25(pg.ml$^{-1}$) de mélatonine :

| Echantillon n° | Rapport de surface de pic de l'extrait de matrice biologique | Etalon non traité |
|---|---|---|
| 1 | 0,169 | 0,221 |
| 2 | 0,173 | 0,208 |
| 3 | 0,173 | 0,213 |
| 4 | 0,187 | 0,210 |
| 5 | 0,179 | 0,211 |
| 6 | 0,169 | 0,211 |
| Moyenne | 0,175 | 0,212 |
| ET | 0,007 | 0,005 |
| n | 6 | 6 |
| ETR (%) | 3,95 % | 2,22 % |
| Valeur minimale | 0,169 | 0,208 |
| Valeur maximale | 0,187 | 0,221 |
| Récupération globale = 82,47 % | | |

Récupération globale calculée d'après les résultats obtenus après l'analyse des répliques d'un extrait de matrice biologique et d'un étalon non traité chargés avec 2(pg.ml$^{-1}$) de mélatonine :

| Echantillon n° | Rapport de surface de pic de l'extrait de matrice biologique | Etalon non traité |
|---|---|---|
| 1 | 0,016 | 0,020 |
| 2 | 0,019 | 0,019 |
| 3 | 0,015 | 0,020 |

(suite)

| Echantillon n° | Rapport de surface de pic de l'extrait de matrice biologique | Etalon non traité |
|---|---|---|
| 4 | 0,018 | 0,021 |
| 5 | 0,018 | 0,020 |
| 6 | 0,018 | 0,019 |
| Moyenne | 0,017 | 0,020 |
| ET | 0,002 | 0,001 |
| n | 6 | 6 |
| ETR (%) | 8,90 % | 3,82 % |
| Valeur minimale | 0,015 | 0,019 |
| Valeur maximale | 0,019 | 0,021 |
| Récupération globale = 87,86 % | | |

Linéarité des courbes d'étalonnage

Les trois courbes d'étalonnage de plasma, obtenues le même jour en traçant les rapports des surfaces des pics de mélatonine/$D_4$-mélatonine en fonction des concentrations connues de mélatonine, étaient des droites (coefficients moyens de corrélation $\pm$ ET = 0,9973 $\pm$ 0,023) sur la plage de concentrations de 1 à 200 pg.ml$^{-1}$. L'analyse de la régression a croisé près de l'origine une valeur moyenne d'ordonnée à l'origine égale à 0,0035 pour n = 3 déterminations (tableau 1). Dans le troisième et le cinquième ensemble d'échantillons témoins de plasma dans le test de sélectivité et de spécificité, le signal moyen observé dans les échantillons de blanc a été équivalent à 8,38 et 1,47 pg.ml$^{-1}$ de mélatonine. A cause de ce taux élevé de mélatonine endogène, ces ensembles d'échantillons témoins de plasma n'ont pas été utilisés pour la procédure de validation subséquente. La valeur d'ETR (1,96 %) calculée à partir de la pente moyenne et l'écart-type associé (0,0068 $\pm$ 0,0001) démontrent la reproductibilité de la technique au cours de la même journée. La linéarité et la reproductibilité d'un jour à l'autre ont été évaluées en utilisant les paramètres de régression de 5 courbes d'étalonnage préparées et exécutées sur une période de 1 semaine par deux analystes différents. Ces courbes d'étalonnage ont été réalisées avec le même ensemble de plasmas témoins. Comme il est montré dans le tableau 2, la valeur moyenne de la pente (0,0069 $\pm$ 0,0001) était proche du «test de linéarité» sur un jour et il y a eu très peu de dispersion des 5 points d'étalonnage le long des tracés de régression (tableau 2).

Tableau 1 : Paramètres de la courbe d'étalonnage au cours de la même journée obtenus lors du « test de linéarité » sur un jour

| Courbe | | Pente | Ordonnée à l'origine | Facteur de détermination |
|---|---|---|---|---|
| 1 | | 0,0069 | 3,3500E-03 | 0,9995 |
| 2 | | 0,0067 | 3,1700E-03 | 0,9992 |
| 3 | | 0,0069 | 4,0800E-03 | 0,9997 |
| | Moyenne | 0,0068 | 0,0035 | 0,9995 |
| | ET | 0,0001 | 0,0006 | 0,0004 |
| | n | 2 | 2 | 2 |
| | ETR (%) | 1,96 % | * | 0,04 % |
| | Valeur minimale | 0,0067 | 3,1700E-03 | 0,9992 |
| | Valeur maximale | 0,0069 | 4,0800E-03 | 0,9997 |

Tableau 2 : Linéarité et reproductibilité d'un jour à l'autre de 5 courbes d'étalonnage de plasma préparées et exécutées sur une période de 1 semaine

| Courbe | | Pente | Ordonnée à l'origine | Facteur de détermination |
|---|---|---|---|---|
| Série 2 | | 0,0070 | 3,7900E-03 | 0,9973 |
| Série 3 | | 0,0068 | 4,8000E-03 | 0,9972 |
| Série 4-1 | | 0,0069 | 3,3500E-03 | 0,9995 |
| Série 4-2 | | 0,0067 | 3,1700E-03 | 0,9992 |
| Série 4-3 | | 0,0069 | 4,1000E-03 | 0,9997 |
| | Moyenne | 0,0069 | 0,0038 | 0,9986 |
| | ET | 0,0001 | 0,0006 | 0,0012 |
| | n | 5 | 5 | 5 |
| | ETR (%) | 1,66 % | * | 0 , 12 % |
| | Valeur minimale | 0,0067 | 0,0032 | 0,9972 |
| | Valeur maximale | 0,0070 | 0,0048 | 0,9995 |

Précision et exactitude

Toutes les sources de variabilité ont été considérablement réduites grâce à l'utilisation d'un analogue d'isotope stable pur en tant qu'étalon interne. Les écarts-types relatifs des différents tests de répétabilité de plasmas calculés sur trois jours distincts ont été inférieurs à 15,2 % et les pourcentages moyens d'erreur ont été dans la plage de -9,71 % à +4,61 % (tableaux 3).

Tableau 3.1. : Précision et exactitude au cours du même jour du procédé de CLHP-MS/MS calculées sur trois jours distincts

| Concentration théorique (pg.ml-1) | n | Concentration expérimentale (pg.ml-1) | Moyenne (pg.ml-1) | ET | ETR (%) | Biais (%) |
|---|---|---|---|---|---|---|
| 1 | 6 | 0,875 1,096 0,834 0,968 1,179 1,143 | 1,016 | 0,145 | 14,23 | 1,58 |
| 2 | 6 | 1,993 1,830 1,634 1,949 1,789 1,641 | 1,806 | 0,150 | 8,33 | -9,71 |
| 25 | 6 | 24,586 24,378 23,413 24,168 24,356 23,706 | 24,101 | 0,450 | 1,87 | -3,60 |
| 200 | 6 | 213,318 209,294 213,664 205,029 | 209,213 | 3,681 | 1,76 | 4,61 |

(suite)

| Concentration théorique (pg.ml⁻¹) | n | Concentration expérimentale (pg.ml⁻¹) | Moyenne (pg.ml⁻¹) | ET | ETR (%) | Biais (%) |
|---|---|---|---|---|---|---|
| | | 208,338 208,633 | | | | |

Tableau 3.2. : Précision et exactitude au cours du même jour du procédé de CLHP-MS/MS calculées sur trois jours distincts

| Concentration théorique (pg.ml⁻¹) | n | Concentration expérimentale (pg.ml⁻¹) | Moyenne (pg.ml⁻¹) | ET | ETR (%) | Biais (%) |
|---|---|---|---|---|---|---|
| 1 | 6 | 1,101 1,292 0,932 0,960 0,897 1,028 | 1,035 | 0,146 | 14,07 | 3,50 |
| 2 | 6 | 1,751 2,225 1,602 1,973 2,029 1,974 | 1,926 | 0,219 | 11,39 | -3,71 |
| 25 | 6 | 23,465 24,003 24,109 26,021 24,938 23,496 | 24,339 | 0,983 | 4,04 | -2,64 |
| 200 | 6 | 199,391 203,061 204,356 198,973 212,388 190,702 | 201,479 | 7,163 | 3,56 | 0,74 |

Tableau 3.3. : Précision et exactitude au cours du même jour du procédé de CLHP-MS/MS calculées sur trois jours distincts

| Concentration théorique (pg.ml⁻¹) | n | Concentration expérimentale (pg.ml⁻¹) | Moyenne (pg.ml⁻¹) | ET | ETR (%) | Biais (%) |
|---|---|---|---|---|---|---|
| 1 | 6 | 1,040 0,887 1,171 1,159 0,796 0,938 | 0,998 | 0,151 | 15,15 | -0,18 |
| 2 | 5 | 1,825 1,730 | 1,906 | 0,200 | 10,50 | -4,72 |

(suite)

| Concentration théorique (pg.ml$^{-1}$) | n | Concentration expérimentale (pg.ml$^{-1}$) | Moyenne (pg.ml$^{-1}$) | ET | ETR (%) | Biais (%) |
|---|---|---|---|---|---|---|
| | | 1,810 *2,883 1,921 2,242 | | | | |
| 25 | 6 | 23,994 24,003 24,221 25,139 24,361 24,827 | 24,424 | 0,465 | 1,90 | -2,30 |
| 200 | 6 | 201,641 208,259 206,438 207,900 206,162 208,489 | 206,482 | 2,559 | 1,24 | 3,24 |

Les résultats des analyses de répétabilité d'un jour à l'autre ont été plutôt semblables à ceux obtenus durant l'analyse au cours du même jour : les ETR ont été inférieurs à 13,68 % et le pourcentage moyen d'erreur s'est situé dans la plage de -6,13 à +2,86 % pour les concentrations de mélatonine testées de 1 à 200 pg.ml 1 (tableau 4).

Tableau 4 : Précision et exactitude d'un jour à l'autre du procédé de CLHP-MS/MS calculées sur une période de 1 semaine durant les analyses de répétabilité

| Concentration théorique (pg. ml$^{-1}$) | n | Moyenne (pg.ml$^{-1}$) | ET | ETR (%) | Biais (%) | ETR moyen (%) |
|---|---|---|---|---|---|---|
| 1 | 18 | 1,016 | 0,139 | 13,68 | 1,64 | 14,48 |
| 2 | 17 | 1,877 | 0,188 | 9,99 | -6,13 | 10,08 |
| 25 | 18 | 24,288 | 0,653 | 2,69 | -2,85 | 2,60 |
| 200 | 18 | 205,724 | 5,645 | 2,74 | 2,86 | 2,18 |

Limites de quantification

Un rapport signal sur bruit > 3 est une valeur encore utilisée par de nombreux auteurs comme critère d'une réponse significative. Malheureusement, même si le bruit électronique est relativement constant d'un jour à l'autre, cela n'est pas toujours vrai pour le bruit de fond chimique, qui est parfois drastiquement différent d'une détermination à une autre. Ceci est principalement dû à l'échantillon lui-même, aux solvants, au matériau d'extraction et au système chromatographique. Afin de prendre en compte toutes ces sources de variabilité, nous avons utilisé une détermination statistique de la limite de quantification. La LOQ théorique, basée sur les résultats obtenus après l'analyse de CLHP-MS/MS de cinq échantillons témoins différents, a été calculée comme étant égale à Yth = 0,25 pg.ml$^{-1}$.

A cause de la présence de mélatonine endogène dans les échantillons de blanc, une analyse de la répétabilité a été réalisée à un taux supérieur égal à 1 pg.ml 1. Un test t de Student a été appliqué pour démontrer que la valeur moyenne du signal YLOQ $\pm$ ET (1,069E-02 $\pm$ 7,405E-04) calculée à partir de l'analyse de la répétabilité réalisée à 1 pg.ml$^{-1}$ a été significativement différente de celle (Ybl) observée avec les 5 échantillons de blanc (2,663E-03 $\pm$ 2,760E-04). Le $t_{cal}$ = -22,5, écart-type = 0,563E-03 pour $n$ = 8 degrés de liberté. La probabilité du résultat, en supposant que l'hypothèse nulle est égale à 0,00. A cause de l'ETR (1,16 %) et du pourcentage moyen d'erreur (-0,40 %) très faibles, cette concentration a été évidemment validée comme la limite de quantification du procédé (tableau 5).

Un chromatogramme de masse type a été enregistré après l'analyse de CLHP-MS/MS d'un plasma témoin. La mélatonine endogène était présente dans cet échantillon à une concentration inférieure à 0,25 pg.ml$^{-1}$. Les chroma-

togrammes de masse des échantillons témoins chargés avec 1 (LLQ) et 200 pg.ml$^{-1}$ de mélatonine ont été obtenus. Lorsqu'un extrait de plasma correspondant à la LLQ du procédé a été analysé, le signal mesuré au temps de rétention de la mélatonine a été équivalent à environ 375 fg injectés dans le système de CLHP-MS/MS. La mélatonine endogène, mesurée dans les échantillons témoins, génère un signal. Il devient évident que des concentrations de mélatonine inférieures à 0,25 pg.ml$^{-1}$ peuvent être quantifiées, durant l'essai clinique, avec une précision et une exactitude élevées comme il est démontré par les résultats de l'analyse de la répétabilité (ETR = 10,36 %) réalisée avec les 5 échantillons de blanc.

Tableau 5 : Procédure de validation pour la détermination de la LOQ au niveau du pg.ml$^{-1}$

| Echantillon n° | Rapport des surfaces de pic de l'extrait des échantillons de blanc | Rapport des surfaces de pic de l'extrait des échantillons de LLQ | Concentration rétrocalculée en pg.ml$^{-1}$ de l'extrait des échantillons de LLQ |
|---|---|---|---|
| 1 | 2,29E-03 | 1,08E-02 | 0,990 |
| 2 | 3,06E-03 | 1,08E-02 | 1,009 |
| 3 | 2,59E-03 | 1,02E-02 | 1,000 |
| 4 | 2,68E-03 | 9,76E-03 | 0,979 |
| 5 | 2,70E-03 | 1,10E-02 | 1,002 |
| Moyenne | $Y_{bl}$ = 2,663E-03 | $Y_{loq}$ = 1,069E-02 | 0,996 |
| ET | 2,760E-04 | 7,405E-04 | 0,012 |
| n | 5 | 5 | 5 |
| ETR (%) | 10,36 % | 6,93 % | 1,16 % |
| Valeur minimale | 2,29E-03 | 9,76E-03 | 0,979 |
| Valeur maximale | 3,06E-03 | 1,17E-02 | 1,009 |

Reproductibilité de l'injection

La reproductibilité de l'étape d'injection a été également étudiée à une concentration (200 pg.ml$^{-1}$) et les résultats de ce test sont résumés dans le tableau 6. Lorsque le même extrait de plasma a été injecté de façon répétée (n = 100), les valeurs d'ETR ont été inférieures à 1,0 %, démontrant ainsi que le procédé était approprié pour une analyse exacte de la mélatonine. En outre, aucun effet mémoire n'a été observé dans l'échantillon de blanc analysé après l'injection de l'extrait de plasma contenant 200 pg.ml 1 de mélatonine.

Tableau 6 : Reproductibilité de l'injection

| Echantillon n° | Temps de rétention de la mélatonine | Surface de pic de la mélatonine | Temps de rétention de l'E.I. | Surtace de pic de l'E.I. | Rapport des surfaces de pic |
|---|---|---|---|---|---|
| Moyenne | 2,33 | 232940 | 2,29 | 370240 | 0,63 |
| ET | 0,01 | 5344 | 0,01 | 10734 | 0,01 |
| ETR (%) | 0,49 % | 2,29 % | 0,49 % | 2,90 % | 0,99 % |
| n | 100 | 100 | 100 | 100 | 100 |
| Valeur minimale | 2,31 | 221000 | 2,28 | 348000 | 0,62 |
| Valeur maximale | 2,36 | 241000 | 2,33 | 385000 | 0,64 |

Test de stabilité

Stabilité durant les cycles de congélation/décongélation

Les résultats de ce test de stabilité (tableaux 7 et 8) ont clairement démontré que les cycles de congélation-décongélation n'ont aucune conséquence sur la stabilité de la mélatonine dans les échantillons de plasma humain stockés à approximativement -20 °C.

Tableau 7 :

| Concentration de référence (pg.ml$^{-1}$) | n | Concentration expérimentale (pg.ml$^{-1}$) | Moyenne (pg. ml$^{-1}$) | ET | ETR (%) | Biais en fonction de la concentration théorique (%) |
|---|---|---|---|---|---|---|
| 1,806 | 6 | 1,862 1,957 2,044 1,812 1,833 1,880 | 1,898 | 0,087 | 4,59 | 5,10 |
| 209,213 | 6 | 208,926 211,252 209,417 219,452 212,865 204,588 | 211,083 | 4,957 | 2,35 | 0,89 |

Tableau 8 : Analyse de la stabilité de la mélatonine après des cycles de congélation/décongélation : précision et biais des mesures de matrice biologique de mélatonine après 3 cycles de congélation/décongélation

| Concentration de référence (pg.ml$^{-1}$) | n | Concentration expérimentale (pg.ml$^{-1}$) | Moyenne (pg.ml$^{-1}$) | ET | ETR (%) | Biais en fonction de la concentration théorique (%) |
|---|---|---|---|---|---|---|
| 1,806 | 5 | 1,889 1,743 1,776 *48,99 2,108 1,960 | 1,895 | 0,147 | 7,78 | 4,96 |
| 209,213 | 6 | 207,575 211,163 203,999 205,980 207,232 212,067 | 208,003 | 3,079 | 1,48 | -0,58 |

Stabilité de l'extrait de plasma

Les résultats rapportés dans le tableau 9 montrent que les extraits des échantillons ont été stables dans le solvant d'injection lorsque les flacons d'injection étaient stockés à approximativement +4°C pendant au moins 24 heures après la première injection pour la mélatonine. Aucune diminution de l'intensité du signal ni aucune modification des tracés chromatographiques n'ont été observées.

Tableau 9 : Stabilité post-préparatoire. Les CQ chargés avec de la mélatonine à deux concentrations (2 et 200 pg.ml 1) ont été stockés sur un auto-échantillonneur et réanalysés (conditions de travail de l'analyse)

| Jour | 2 pg.ml$^{-1}$ | 200 pg.ml$^{-1}$ |
|---|---|---|
| T0 (série 2) | 1,751 | 199,391 |
| | 2,225 | 203,061 |
| | 1,602 | 204,356 |
| | 1,973 | 198,973 |
| | 2,029 | 212,388 |
| | 1,974 | 190,702 |
| Moyenne | 1,926 | 201,479 |
| | | |
| T0 + 24 h (série 3) | 1,817 | 201,388 |
| | 1,908 | 213,337 |
| | 1,800 | 205,171 |
| | 2,560 | 207,755 |
| | 1,806 | 208,103 |
| | 1,854 | 213,387 |
| Moyenne | 1,958 | 208,190 |
| Ecart T0 + 24 h/T0 | 1,65 % | 3,33 % |

3.2 Calcul du rapport entre les doses de VLT et de 6-MH administrées dans l'association, pendant 8 heures, à partir de 22h (ou au coucher), qui permet d'assurer la prévalence des concentrations de VLT, dans l'organisme pendant la période de repos (entre 22h et 6h), puis la prévalence des concentrations de 6-MH à partir de 6h (ou du lever) pendant la période d'activité jusqu'à 22H.

Afin de garantir la prévalence des concentrations de la VLT dans l'organisme, pendant la période de repos entre 22h (ou à l'heure du coucher) et 6h (ou à l'heure du lever), puis celle du 6-MH tout au long de la période d'activité, entre le lever et le coucher, il faut injecter dans l'organisme, pendant la durée d'application du patch, des doses de VLT et de 6-MH dont les valeurs doivent être dans un rapport Dose biodisponible de VLT / Dose biodisponible de 6-MH, qui assure les prévalences et leur alternance pendant toute la durée du nycthémère. Le calcul de ce rapport entre les doses biodisponibles peut être fait à partir de leurs paramètres pharmacocinétiques, de la façon ci-après indiquée.

En fixant une dose biodisponible égale à 40 μg de VLT, dose médiane qui nous paraît adaptée au traitement des insomnies primaires et des affections neurodégénératives du type Parkinson, correspondant à une sécrétion pinéale de MLT faible (sécrétion inférieure à 5 μg de MLT, et concentration plasmatique de MLT à partir de 1h du matin inférieure à 10 pg/ml), on peut calculer la dose biodisponible de 6-MH à co-administrer pour assurer les prévalences des deux hormones et leurs alternances pendant les périodes de repos et d'activité.

Lors d'une infusion de VLT (ou de 6-MH) à vitesse constante (ordre 0 en pharmacocinétique) dans la circulation sanguine, les concentrations plasmatiques de VLT augmentent progressivement jusqu'à ce qu'elles atteignent un plateau, lorsque la vitesse d'élimination devient égale à la vitesse d'infusion. Dès lors, les entrées de VLT sont égales aux sorties ; et les concentrations plasmatiques sont maintenues à une valeur constante. C'est l'état de « plateau ». Si la quantité infusée de VLT, sur une période de 8 heures, est égale à 40 μg, nous pouvons écrire à l'état de plateau :

```
Relation 1 :

    Vitesse d'infusion(de VLT)= 40μg/8h = Vitesse d'élimination(de VLT)= CLxC_SS,
```

relation dans laquelle Cl est la clairance totale (ou clairance plasmatique, exprimée en litres par heure, 1/h) ;
avec CL = (0,693/T$_{1/2}$z) x V$_d$ss,
relation dans laquelle V$_d$ss est le volume de distribution à l'état d'équilibre (steady state : ss), qui correspond à l'état de plateau, à partir duquel les concentrations plasmatiques de VLT n'augmentent plus (à partir de 1h du matin), demeurent en plateau (steady state), à leur valeur maximale $C_{SS}$.

L'étude pharmacocinétique de la VLT et du 6-MH réalisée chez le chien, après administration intraveineuse d'une association de VLT (3mg/kg) et de 6-MH (1mg/kg) a permis de déterminer les valeurs des paramètres pharmacocinétiques, présentées ci-après :

VLT :

$T_{1/2}z$ = 0,70h
$V_d$ss = 5 litres/kg de poids corporel, soit 320l pour un poids corporel de 64 kg chez l'homme.

6-MH :

$T_{1/2}z$ = 2,27h
$V_d$ss = 15 litres/kg de poids corporel, soit 960l pour un poids corporel de 64 kg chez l'homme.

Comme les profils pharmacocinétiques, c'est-à-dire les valeurs des paramètres pharmacocinétiques, sont identiques chez l'homme et chez le chien, de même que chez tous les animaux à sommeil nocturne, il est possible d'extrapoler à l'homme les valeurs des paramètres PK observées chez le chien.

Les prévalences et les alternances des concentrations des deux hormones pendant les périodes de repos et d'activité, doivent être assurées non seulement au niveau du sang (concentrations plasmatiques), mais aussi au niveau de leurs lieux d'action (sites récepteurs 5-HT$_{2C}$, $\alpha_2$, D$_1$ et D$_2$, etc). Il faut donc tenir compte des valeurs des volumes de distribution ($V_d$ss) des deux hormones, qui relient les concentrations plasmatiques des hormones à leurs concentrations au niveau de leurs lieux d'action.

Calcul de la concentration plasmatique maximale ($C_{SS}$) de VLT pour une quantité infusée de VLT égale à 40 $\mu$g chez l'homme. D'après la rlation 1, précédemment énoncée, on a pu écrire :

$$C_{SS} \ (VLT) \ = \ \frac{40\,\mu g \ . \ 0,70\,h}{0,693 \ . \ 8\,h \ . \ 320l} \ = \ 15,8$$ picogrammes par ml, pour la VLT.

Pour le 6-MH qui a un volume de distribution 3 fois plus élevé que celui du VLT, les concentrations plasmatiques maximales $C_{SS}$ devront être maintenues à des valeurs 3 fois plus faibles que celles de la VLT, pour maintenir la prévalence des concentrations de 6-MH, pendant la période de repos, à la fois dans le plasma et au niveau périphérique des sites récepteurs. A cette fin, la dose infusée de 6-MH, associée à une dose infusée de 40 $\mu$g de VLT, ne doit pas dépasser 10 $\mu$g. En effet, pour une dose infusée de 10 $\mu$g, nous avons :

$$C_{SS}(6-MH) \ = \ \frac{10\mu g \ . \ 2,27h}{0,693 \ . \ 8h \ . \ 960l} \ = \ 4,3$$ picogrammes par ml, pour le 6-MH.

Compte tenu d'un volume de distribution 3 fois supérieur à celui de la VLT, les concentrations maximales de 6-MH au voisinage des récepteurs seront toujours inférieures à celles de la VLT, pendant la période de repos.

En conclusion, pour garantir les prévalences des deux hormones (VLT et 6-MH) et leurs alternances, il faut infuser dans la circulation sanguine des doses de VLT au moins quatre fois plus élevées que celles du 6-MH.

Selon un autre aspect particulier de la présente invention, le système thérapeutique transdermique adhésif contient une association d'une charge de valentonine (VLT) et de 6-méthoxy-harmalan (6-MH) susceptible de délivrer dans la circulation sanguine une association de valentonine et de 6-méthoxy-harmalan dans une proportion massique [VLT] / [6-MH] au moins égale à 4 pendant la durée d'application du patch, de préférence pendant environ 8h à 10h.

Quant au niveau, c'est-à-dire l'ordre de grandeur des doses, nous nous sommes basés sur les valeurs des sécrétions pinéales de mélatonine mesurées chez l'homme.

Pour le traitement des troubles du sommeil ainsi que des dépressions nerveuses endogènes et réactionnelles, affections caractérisée par des concentrations plasmatiques maximales de MLT, mesurées chez les patients à partir de 1h du matin, comprises entre 10 pg/ml et 50 pg/ml, il est préconisé d'appliquer un patch au moment du coucher (vers 22h ou plus tard selon les habitudes), avec une charge en VLT et 6-MH permettant de délivrer à l'organisme des doses égales à 20 $\mu$g de VLT et 5 $\mu$g de 6-MH, pendant la durée d'application du patch, comprise entre 8h et 10h, selon l'heure du lever, à laquelle le patch doit être enlevé.

Selon un autre aspect particulier de l'invention, le système thérapeutique transdermique adhésif, contient une charge en VLT et en 6-MH permettant de délivrer dans la circulation sanguine une dose d'au moins 20 $\mu$g de VLT et

d'au moins 5 µg de 6-MH pendant la durée de d'application du patch, de préférence pendant environ 8h à 10h, pour son utilisation dans le traitement des troubles du sommeil et des dépressions nerveuses.

Il est préconisé d'appliquer un patch au moment du coucher (vers 22h, ou plus tard selon les habitudes), avec une charge en VLT et 6-MH permettant de délivrer à l'organisme des doses égales à 40 µg de VLT et 10 µg de 6-MH pendant la durée d'application du patch, comprise entre 8h et 10h, selon l'heure du lever, à laquelle le patch doit être enlevé, pour son utilisation dans le traitement des insomnies primaires et des affections neurodégénératives telles que la maladie de Parkinson, affections caractérisées par des concentrations plasmatiques maximales de MLT, mesurées chez les patients à partir de 1h du matin, inférieures à 10 pg/ml.

Selon une autre caractéristique de l'invention, le système thérapeutique transdermique adhésif contient une charge en VLT et en 6-MH permettant de délivrer dans la circulation sanguine une dose d'au moins 40 µg de VLT et d'au moins 10 µg de 6-MH pendant la durée d'application du patch, de préférence pendant environ 8h à 10h, pour son utilisation dans le traitement d'insomnies primaires et/ou d'affections du type maladie de Parkinson.

Il est préconisé d'appliquer un patch au moment du coucher (vers 22h, ou plus tard selon les habitudes), avec une charge en VLT et 6-MH, pendant la durée d'application du patch, comprise entre 8h et 10h selon l'heure du lever à laquelle le patch doit être enlevé, pour son utilisation dans le traitement des affections neurodégénératives du type de la maladie d'Alzheimer, affections caractérisées par des concentrations plasmatiques maximales de MLT, mesurée chez les patients à partir de 1h du matin, inférieures à lpg/ml, voire non mesurables.

Selon un autre aspect particulier de l'invention, le système thérapeutique transdermique adhésif, contient une charge en VLT et en 6-MH permettant de délivrer dans la circulation sanguine une dose d'au moins 60 µg de VLT et d'au moins 15 µg de 6-MH pendant la durée d'application du patch, de préférence pendant environ 8h à 10h, pour son utilisation destinée à la prévention et/ou au traitement de la maladie d'Alzheimer.

Comme déjà indiqué précédemment, la présente invention s'étend également à son utilisation destinée à la prévention et/ou au traitement des affections dues à une sécrétion pinéale excessive des hormones du système [(VLT)-(6-MH)]. Le cas particulier de son utilisation destinée au traitement des psychoses consiste à appliquer, pendant la période d'activité, un patch chargé uniquement en valentonine. La VLT doit permettre de déplacer l'excès de 6-méthoxy-harmalan sécrété chez les patients psychotiques.

## Revendications

1.  Système thérapeutique transdermique adhésif, **caractérisé en ce qu'**il contient à titre de principe actif une association de valentonine (VLT) et de 6-méthoxy-harmalan (6-MH).

2.  Système thérapeutique transdermique adhésif selon la revendication précédente, **caractérisé en ce qu'**il contient une association d'une charge de valentonine (VLT) et de 6-méthoxy-harmalan (6-MH) apte à délivrer dans la circulation sanguine la valentonine et de 6-méthoxy-harmalan dans une proportion massique [VLT] / [6-MH] au moins égale à 4 pendant la durée d'application du patch, de préférence pendant environ 8h à 10h.

3.  Système thérapeutique transdermique adhésif selon les revendications 1 et 2 pour son utilisation à titre de médicament.

4.  Système thérapeutique transdermique adhésif, selon l'une des revendications 1 à 3 contenant une charge en VLT et en 6-MH permettant de délivrer dans la circulation sanguine une dose d'au moins 20 µg de VLT et d'au moins 5 µg de 6-MH pendant la durée d'application du patch, de préférence pendant environ 8h à 10h, pour son utilisation dans le traitement des troubles du sommeil et des dépressions nerveuses.

5.  Système thérapeutique transdermique adhésif, selon l'une des revendications 1 à 3 contenant une charge en VLT et en 6-MH permettant de délivrer dans la circulation sanguine une dose d'au moins 40 µg de VLT et d'au moins 10 µg de 6-MH pendant la durée d'application du patch, de préférence pendant environ 8h à 10h, pour son utilisation dans le traitement d'insomnies primaires et/ou d'affections du type maladie de Parkinson.

6.  Système thérapeutique transdermique adhésif, selon l'une des revendications 1 à 3 contenant une charge en VLT et en 6-MH permettant de délivrer dans la circulation sanguine une dose d'au moins 60 µg de VLT et d'au moins 15 µg de 6-MH pendant la durée d'application du patch, de préférence pendant environ 8h à 10h, pour son utilisation destinée à la prévention et/ou au traitement de la maladie d'Alzheimer.

**Patentansprüche**

1. Adhäsives transdermales therapeutisches System, **dadurch gekennzeichnet, dass** es als Wirkprinzip eine Kombination aus Valentonin (VLT) und 6-Methoxy-harmalan (6-MH) enthält.

2. Adhäsives transdermales therapeutisches System nach vorangehendem Anspruch, **dadurch gekennzeichnet, dass** es eine Kombination einer Charge aus Valentonin (VLT) und 6-Methoxy-harmalan (6-MH) enthält, die imstande ist, in den Blutkreislauf Valentonin und 6-Methoxy-harmalan in einem Massenverhältnis [VLT] / [6-MH] von mindestens gleich 4 während der Anwendungsdauer des Patchs von vorzugsweise während zirka 8 Stunden bis 10 Stunden abzugeben.

3. Adhäsives transdermales therapeutisches System nach den Ansprüchen 1 und 2 für seine Verwendung als Arzneimittel.

4. Adhäsives transdermales therapeutisches System nach einem der Ansprüche 1 bis 3, enthaltend eine Charge aus VLT und 6-MH, die erlaubt, in den Blutkreislauf eine Dosis von mindestens 20 $\mu$g VLT und von mindestens 5 $\mu$g 6-MH während der Anwendungsdauer des Patchs vorzugsweise während zirka 8 Stunden bis 10 Stunden seine Verwendung bei der Behandlung von Schlafstörungen und nervösen Depressionen abzugeben.

5. Adhäsives transdermales therapeutisches System nach einem der Ansprüche 1 bis 3, enthaltend eine Charge aus VLT und 6-MH, die erlaubt, in den Blutkreislauf eine Dosis von mindestens 40 $\mu$g VLT und von mindestens 10 $\mu$g 6-MH während der Anwendungsdauer des Patchs vorzugsweise während zirka 8 Stunden bis 10 Stunden für seine Verwendung bei der Behandlung von primärer Schlaflosigkeit und/oder Erkrankungen von Typ Parkinson abzugeben.

6. Adhäsives transdermales therapeutisches System nach einem der Ansprüche 1 bis 3, enthaltend eine Charge aus VLT und 6-MH, die erlaubt, in den Blutkreislauf eine Dosis von mindestens 60 $\mu$g VLT und von mindestens 15 $\mu$g 6-MH während der Anwendungsdauer des Patchs vorzugsweise während zirka 8 Stunden bis 10 Stunden für seine Verwendung bei der Vorbeugung und/oder Behandlung von Alzheimer abzugeben.

**Claims**

1. An adhesive transdermal therapeutic system, **characterized in that** it contains, as an active ingredient, an association of Valentonin (VLT) and of 6-methoxy-harmalan (6-MH).

2. The adhesive transdermal therapeutic system according to the preceding claim, **characterized in that** it contains an association of a charge of Valentonin (VLT) and of 6-methoxy-harmalan (6-MH) able to deliver, into the bloodstream, the Valentonin and 6-methoxy-harmalan in a mass proportion [VLT] / [6-MH] at least equal to 4 during the period of application of the patch, preferably for about 8h to 10h.

3. The adhesive transdermal therapeutic system according to claims 1 and 2 for use thereof as a medicine.

4. The adhesive transdermal therapeutic system, according to any of claims 1 to 3 containing a charge in VLT and in 6-MH for delivering, into the bloodstream, a dose of at least 20 $\mu$g of VLT and at least 5 $\mu$g of 6-MH during the period of application of the patch, preferably for about 8h to 10h, for use thereof in the treatment of sleep disturbances and nervous breakdowns.

5. The adhesive transdermal therapeutic system, according to any of claims 1 to 3 containing a charge in VLT and in 6-MH for delivering, into the bloodstream, a dose of at least 40 $\mu$g of VLT and at least 10 $\mu$g of 6-MH during the period of application of the patch, preferably for about 8h to 10h, for use thereof in the treatment of primary insomnia and/or disorders such as Parkinson disease.

6. The adhesive transdermal therapeutic system, according to any of claims 1 to 3 containing a charge in VLT and in 6-MH for delivering, into the bloodstream, a dose of at least 60 $\mu$g of VLT and at least 15 $\mu$g of 6-MH during the period of application of the patch, preferably for about 8h to 10h, for use thereof in the prevention and/or treatment of Alzheimer's disease.

## RÉFÉRENCES CITÉES DANS LA DESCRIPTION

**Documents brevets cités dans la description**

- FR 2898358

**Littérature non-brevet citée dans la description**

- **FOURTILLAN JB ; BRISSON A.M. ; GOBIN P. ; FOURTILLAN M. ; INGRAND I. ; DECOURT J.PH. ; GIRAULT J.** Melatonin secretion occurs at a constant rate in both young and older men and women. *Am, J. Physiol. Endocrinol. Metab.,* 2001, vol. 280, E11-E22

- *Am.J.Physiol.Endocrinol.metab.,* vol. 280, E11-E22